(19) Europäisches Patentamt
European Patent Office
Office européen des brevets

(11) Numéro de publication: **0 110 795**
**B1**

# (12) FASCICULE DE BREVET EUROPEEN

(45) Date de publication du fascicule du brevet: **12.03.86**

(51) Int. Cl.⁴: **C 07 D 207/24**

(21) Numéro de dépôt: **83402354.1**

(22) Date de dépôt: **06.12.83**

(54) **Nouveaux dérivés de la pyrrolidine, leur préparation et les médicaments qui les contiennent.**

(30) Priorité: **07.12.82 FR 8220492**

(43) Date de publication de la demande:
**13.06.84 Bulletin 84/24**

(45) Mention de la délivrance du brevet:
**12.03.86 Bulletin 86/11**

(84) Etats contractants désignés:
**AT BE CH DE FR GB IT LI LU NL SE**

(56) Documents cités:
**US - A - 4 247 549**

**CHEMICAL ABSTRACTS, vol. 94, no. 4, 26 janvier 1981, page 570, no. 30707u, Columbus, Ohio, US S. ZIKOLOVA et al.: "Synthesis of piperazine derivatives. XIII. Preparation of N1-substituted N4-(chloroacetyl)piperazines"**

(73) Titulaire: **RHONE-POULENC SANTE, Les Miroirs 18 Avenue d'Alsace, F-92400 Courbevoie Cedex (FR)**

(72) Inventeur: **Fabre Jean-Louis, 9, rue Fagon, F-75013 Paris (FR)**
Inventeur: **Farge, Daniel, 30, rue des Pins Sylvestres, F-94320 Thiais (FR)**
Inventeur: **James, Claude, 31 bis, avenue Gambetta, F-75020 Paris (FR)**
Inventeur: **Lavé, Daniel, 72, Bld de la Villette, F-75019 Paris (FR)**

(74) Mandataire: **Le Goff, Yves et al, RHONE-POULENC RECHERCHES Brevets Pharma 25, Quai Paul Doumer, F-92408 Courbevoie (FR)**

**EP 0 110 795 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la pyrrolidine de formule générale:

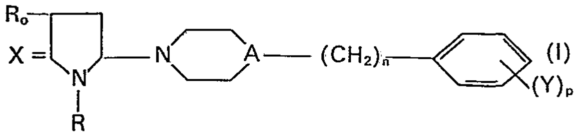
(I)

leurs sels, leur préparation et les compositions médicinales qui les contiennent.

Dans la formule générale (I), X représente un atome d'oxygène ou de soufre, R représente un atome d'hydrogène ou un radical alcoyle, $R_o$ représente un atome d'hydrogène ou un radical alcoyle ou phényle éventuellement substitué par un atome d'halogène ou par un radical alcoyle, alcoyloxy, alcoylthio ou nitro, A représente un atome d'azote ou un radical =CH−, les symboles Y, identiques ou différents, représentent un atome d'hydrogène ou d'halogène ou un radical alcoyle, alcoylthio, alcoylcarbonyle, nitro, amino, alcoylcarbonylamino, alcoylamino, dialcoylamino, alcoylsulfinyle, alcoylsulfonyle, alcoyloxycarbonyle, cyano, trifluorométhyle, hydroxy, mercapto, alcoylcarbonyloxy ou alcoylcarbonylthio, n représente le nombre 0 ou 1 et p représente le nombre 1, 2 ou 3, étant entendu que dans les définitions précédentes et celles qui seront données par la suite, tous les radicaux alcoyle et portions alcoyle sont en chaîne droite ou ramifiée et contiennent 1 à 4 atomes de carbone.

Selon l'invention, les produits de formule générale (I) dans laquelle X représente un atome d'oxygène et les autres symboles sont définis comme précédemment peuvent être obtenus par action d'un produit de formule générale:

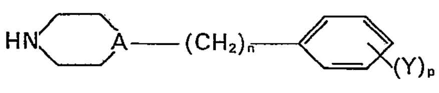
(II)

dans laquelle A, Y, n et p sont définis comme précédemment sur une pyrrolidinone-2 de formule générale:

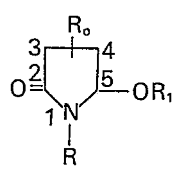
(III)

dans laquelle R et $R_o$ sont définis comme précédemment et $R_1$ représente un radical alcoyle (de préférence éthyle).

On opère généralement sans solvant à une température comprise entre 70 et 180° C ou dans un solvant inerte tel qu'un carbure aromatique (xylène) à la température de reflux du mélange réactionnel, en éliminant éventuellement l'alcool formé.

Selon la nature du substituant Y, il peut être avantageux de protéger la fonction dudit substituant avant d'opérer la condensation sur la pyrrolidinone-2 de formule générale (III), la fonction étant libérée après la condensation. Le blocage et le déblocage peuvent s'effectuer selon les méthodes habituelles connues de l'homme du métier et qui n'affectent pas le reste de la molécule.

Les produits de formule générale (III) peuvent être obtenus par réduction de la pyrrolidinedione-2,5 de formule générale:

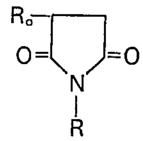
(IV)

dans laquelle R et $R_o$ sont définis comme précédemment, en opérant dans un alcool de formule générale $R_1OH$ dans laquelle $R_1$ est défini comme précédemment.

On effectue la réduction au moyen d'un borohydrure alcalin en présence d'acide chlorhydrique anhydre comme décrit par J.C. Hubert, J.B.P.A. Wijnberg et W.N. Speckamp, «Tetrahedron», *31*, 1437 (1975).

Lorsque $R_o$ est différent d'un atome d'hydrogène, la réduction s'effectue indifféremment sur l'une ou l'autre des fonctions carbonyle de la pyrrolidinedione de formule générale (IV) et l'on obtient le produit de formule générale (III) sous forme d'un mélange de produits dans lesquels $R_o$ se trouve en position 3 ou en position 4. Généralement le mélange peut être utilisé tel quel sans inconvénient dans la synthèse ultérieure; son emploi ne nuit pas à la purification du produit de formule générale (I).

Selon l'invention, les produits de formule générale (I) dans laquelle $R_o$, A, Y, n et p sont définis comme précédemment, A représente un atome d'oxygène et R représente un radical alcoyle, peuvent avantageusement être préparés par condensation d'un produit de formule générale:

$$R'Z \qquad (V)$$

dans laquelle R' représente un radical alcoyle et Z représente un atome d'halogène ou un reste d'ester réactif tel que mésyloxy ou tosyloxy sur un produit de formule générale (I) dans laquelle $R_o$, A, Y, n et p sont définis comme précédemment et R représente un atome d'hydrogène, c'est-à-dire un produit de formule générale:

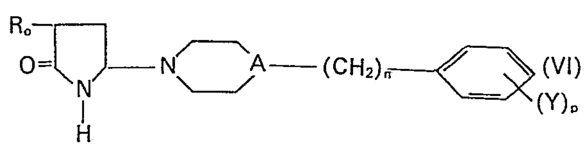
(VI)

On opère généralement dans un solvant organique anhydre tel que le diméthylformamide en présence d'un agent de condensation tel qu'un hydrure alcalin, à une température comprise entre 0 et 60° C. Si le substituant Y possède une fonction qui peut être alcoylée, il est avantageux de bloquer préalablement la fonction dudit substituant avant de faire réagir le produit de formule générale (V). Le blocage et le déblocage subséquent s'effectuent selon les méthodes habituelles connues de l'homme du métier et qui n'affectent pas le reste de la molécule.

Selon l'invention, les produits de formule générale (I) dans laquelle X représente un atome de soufre et les autres symboles sont définis comme précédemment peuvent être obtenus par thionation d'un produit de formule générale (I) dans laquelle X représente un atome d'oxygène et les autres symboles sont définis comme précédemment, c'est-à-dire un produit de formule générale:

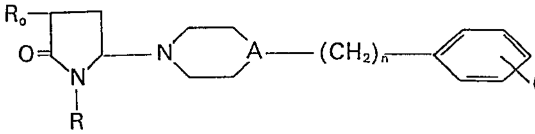

(VII)

La thionation s'effectue généralement au moyen de pentasulfure de phosphore dans un solvant organique tel que le toluène, le dioxanne ou le diméthoxy-1,2 éthane, à une température voisine de 100° C ou mieux au moyen du réactif de Lawesson, bis(méthoxy-4 phényl)-2,4 dithioxo-2,4 dithia-1,3 diphospha-2,4 étanne, dans un solvant organique tel que le toluène à une température voisine de 50° C ou le diméthoxy-1,2 éthane ou l'hexaméthylphosphoramide à une température voisine de 20° C. Lorsque le substituant Y possède une fonction sensible à la thionation, cette fonction devra être protégée au préalable par un groupement protecteur labile selon les techniques habituelles connues de l'homme du métier.

Les nouveaux produits de formule générale (I) peuvent être purifiés par les méthodes habituelles telles que cristallisation, chromatographie, ou extractions successives en milieu acide ou basique.

Les nouveaux produits de formule générale (I) peuvent être transformés éventuellement en sels d'addition avec les acides par réaction d'un acide dans un solvant organique tel qu'un alcool, une cétone, un ester ou un solvant chloré; le sel formé précipite, éventuellement après concentration de sa solution. Il est séparé par filtration ou décantation.

Les nouveaux produits de formule générale (I) et leurs sels présentent d'intéressantes propriétés pharmacologiques les rendant utiles comme antidépresseurs.

Ils se sont montrés actifs en particulier dans le test de l'action antagoniste vis-à-vis de la dépression induite par la tétrabénazine chez le rat à des doses comprises entre 1 et 100 mg/kg par voie sous-cutanée ou orale.

Leur dose létale $DL_{50}$ est généralement comprise entre 100 et 900 mg/kg chez la souris par voie orale.

On connaît par le brevet américain N° 4247549 des esters d'acide pipérazinecarboxylique-1 possédant des activités antidépressives et analgésiques. Toutefois ces produits ne suggèrent pas les produits de la présente invention. De plus, les produits de la présente invention présentent des propriétés antidépressives plus marquées.

Sont d'un intérêt particulier les produits de formule générale (I) dans laquelle X représente un atome d'oxygène ou de soufre, $R_0$ représente un atome d'hydrogène, R représente un atome d'hydrogène ou un radical alcoyle, A représente un atome d'azote ou un radical =CH−, Y représente un atome d'hydrogène ou d'halogène ou un radical alcoyle, alcoyloxy, alcoylcarbonyle, nitro ou trifluorométhyle, n est égal à 0 ou 1 et p est égal à 1.

Sont plus particulièrement intéressants les produits de formule générale (I) dans laquelle X représente un atome d'oxygène, $R_0$ et R représentent un atome d'hydrogène, A représente un atome d'azote, Y représente un atome d'hydrogène ou d'halogène ou un radical alcoyle, alcoyloxy, alcoylcarbonyle ou nitro, n est égal à 0 et p est égal à 1.

Pour l'emploi médicinal, il peut être fait usage des nouveaux produits de formule générale (I) tels quels ou, le cas échéant, à l'état de sels pharmaceutiquement acceptables, c'est-à-dire non toxiques aux doses d'utilisation.

Comme exemples de sels pharmaceutiquement acceptables peuvent être cités les sels d'addition avec les acides minéraux (tels que chlorhydrates, sulfates, nitrates, phosphates) ou organiques (tels que les acétates, propionates, succinates, benzoates, fumarates, maléates, méthanesulfonates, iséthionates, théophyllineacétates, salicylates, phénolphtalinates, méthylène-bis-β-oxynaphtoates) ou des dérivés de substitution de ces composés.

Les exemples suivants, donnés à titre non limitatif, montrent comment l'invention peut être mise en pratique.

*Exemple 1:*

Un mélange de 10,4 g d'éthoxy-5 pyrrolidinone-2 et de 19,4 g de phényl-4 pipérazine est chauffé sous agitation pendant 1 h et 15 min à une température comprise entre 124 et 130° C. Après refroidissement du mélange réactionnel, on ajoute 30 $cm^3$ d'éthanol pour mettre en suspension le solide obtenu. Les cristaux sont séparés par filtration, lavés 3 fois par 30 $cm^3$ au total d'éthanol puis 3 fois par 75 $cm^3$ au total d'éther diéthylique et séchés sous pression réduite (20 mm de mercure; 2,7 kPa) à une température voisine de 20° C en présence de potasse en pastilles. On obtient ainsi 15,8 g de produit brut fondant à 206° C. Ce produit est dissous dans 350 $cm^3$ de méthanol bouillant; la solution est additionnée de 1 g de noir décolorant et filtrée à chaud. Le filtrat est refroidi à une température voisine de 4° C pendant 1 h. Les cristaux apparus sont séparés par filtration, lavés 3 fois par 75 $cm^3$ au total de méthanol puis 3 fois par 75 $cm^3$ au total d'éther diéthylique et séchés sous pression réduite (20 mm de mercure; 2,7 kPa) à une température voisine de 20° C en présence de potasse en pastilles. On obtient ainsi 12,1 g de (phényl-4 pipérazinyl-1)-5 pyrrolidinone-2 sous forme de cristaux blancs fondant à 210° C.

L'éthoxy-5 pyrrolidinone-2 peut être préparée comme décrit par J.C. Hubert, J.B.P.A. Wijnberg et W.N. Speckamp «Tetrahedron», *31*, 1437, (1975).

*Exemple 2:*

Un mélange de 7,75 g d'étoxy-5 pyrrolidinone-2 et de 10,6 g de (méthyl-2 phényl)-1 pipérazine est chauffé sous agitation pendant 2 h à une température comprise entre 127 et 140° C en distillant l'éthanol formé. Après refroidissement du mélange réactionnel, le solide obtenu est séparé par filtration; lavé 4 fois par 35 $cm^3$ au total d'acétate d'éthyle et séché sous pression réduite (20 mm de mercure; 2,7 kPa) à une température voisine de 20° C en présence de potasse en pastilles. On obtient ainsi 12,45 g de produit brut. Ce produit est dissous dans 150 $cm^3$ d'acétonitrile bouillant; la solution est additionnée de 1 g de noir décolorant et filtrée à chaud. Le filtrat est refroidi à une température voisine de 4° C pendant 16 h. Les cristaux apparus sont séparés par filtration, lavés 3 fois par 30 $cm^3$ au total d'acétonitrile refroidi à une température voisine de 4° C et séchés sous pression réduite (20 mm de mercure; 2,7 kPa) à une température voisine de 20° C en présence de potasse en pastilles. On obtient ainsi 9,95 g de [(méthyl-2 phényl)-4 pipérazinyl-1]-5 pyrrolidinone-2 sous forme de cristaux blancs fondant à 166° C.

*Exemple 3:*

Un mélange de 12,9 g d'étoxy-5 pyrrolidinone-2 et de 18 g de (fluoro-4 phényl)-1 pipérazine est chauffé sous agitation pendant 45 min à une température comprise entre 155 et 169° C en distillant l'éthanol formé. Après refroidissement du mélange réactionnel, le solide obtenu est séparé par filtration, lavé 4 fois par 40 $cm^3$ au total d'acétate d'éthyle et séché sous pression réduite (20 mm de mercure; 2,7 kPa) à une température voisine de 20° C en présence de potasse en pastilles. On obtient ainsi 20,1 g de produit brut fondant à 170° C. Ce produit est dissous dans 800 $cm^3$ d'acétate d'éthyle bouillant; la solution est additionnée de 1 g de noir décolorant et filtrée à chaud. Le filtrat est refroidi à une température voisine de 4° C pendant 3 d. Les cristaux apparus sont séparés par filtration, lavés 2 fois par 20 $cm^3$ au total d'acétate d'éthyle et séchés sous pression réduite (20 mm de mercure; 2,7 kPa) à une température voisine de 20° C en présence de potasse en pastilles. On obtient ainsi 17,6 g de [(fluoro-4 phényl)-4 pipérazinyl-1]-5 pyrrolidinone-2 sous forme de cristaux blancs fondant à 180° C.

*Exemple 4:*

Un mélange de 9,6 g d'étoxy-5 pyrrolidinone-2 et de 14,7 g de (chloro-4 phényl)-1 pipérazine est chauffé sous agitation pendant 1 h et 15 min à une température comprise entre 127 et 136° C en distillant l'éthanol formé. Après refroidissement du mélange réactionnel, le solide obtenu est séparé par filtration, lavé 3 fois par 25 $cm^3$ au total d'acétate d'éthyle et séché sous pression réduite (20 mm de mercure; 2,7 kPa) à une température voisine de 20° C en présence de potasse en pastilles. On obtient ainsi 15,35 g de produit brut fondant à 202° C. Ce produit est dissous dans 650 $cm^3$ d'éthanol bouillant; la solution est additionnée de 1 g de noir décolorant et filtrée à chaud. Le filtrat est refroidi à une température voisine de 4° C pendant 3 h. Les cristaux apparus sont séparés par filtration, lavés 3 fois par 150 $cm^3$ au total d'éthanol refroidi à une température voisine de 4° C et séchés sous pression réduite (20 mm de mercure; 2,7 kPa) à une température voisine de 20° C en présence de potasse en pastilles. On obtient ainsi 12,5 g de [(chloro-4 phényl)-4 pipérazinyl-1]-5 pyrrolidinone-2 sous forme de cristaux blancs fondant à 209° C.

*Exemple 5:*

Un mélange de 7,75 g d'étoxy-5 pyrrolidinone-2 et de 11,8 g de (chloro-3 phényl)-1 pipérazine est chauffé sous agitation pendant 2 h et 35 min à une température comprise entre 136 et 140° C en distillant l'éthanol formé. Après refroidissement du mélange réactionnel, le solide obtenu est séparé par filtration, lavé 3 fois par 30 $cm^3$ au total d'acétate d'éthyle puis 2 fois par 50 $cm^3$ au total d'éther diéthylique et séché sous pression réduite (20 mm de mercure; 2,7 kPa) à une température voisine de 20° C en présence de potasse en pastilles. On obtient ainsi 13,3 g de produit brut.

Ce produit est dissous dans 105 $cm^3$ d'éthanol bouillant; la solution est additionnée de 1 g de noir décolorant et filtrée à chaud. Le filtrat est refroidi à une température voisine de 4° C pendant 16 h. Les cristaux apparus sont séparés par filtration, lavés 3 fois par 30 $cm^3$ au total d'éthanol refroidi à une température voisine de 4° C et séchés sous pression réduite (20 mm de mercure; 2,7 kPa) à une température voisine de 20° C en présence de potasse en pastilles. On obtient ainsi 11,15 g de [(chloro-3 phényl)-4 pipérazinyl-1]-5 pyrrolidinone-2 sous forme de cristaux beiges fondant à 158° C.

*Exemple 6:*

Un mélange de 7,75 g d'étoxy-5 pyrrolidinone-2 et de 11,8 g de (chloro-2 phényl)-1 pipérazine est chauffé sous agitation pendant 1 h à une température comprise entre 137 et 145° C en distillant l'éthanol formé. Après refroidissement du mélange réactionnel, le solide obtenu est séparé par filtration, lavé 4 fois par 40 $cm^3$ au total d'acétate d'éthyle et séché sous pression réduite (20 mm de mercure; 2,7 kPa) à une température voisine de 20° C en présence de potasse en pastilles. On obtient ainsi 12,45 g de produit brut fondant à 192° C. Ce produit est dissous dans 260 $cm^3$ d'éthanol bouillant; la solution est additionnée de 1 g de noir décolorant et filtrée à chaud. Le filtrat est refroidi à une température voisine de 4° C pendant 2 h. Les cristaux apparus sont séparés par filtration, lavés 2 fois par 20 $cm^3$ au total d'éthanol refroidi à une température voisine de 4° C et séchés sous pression réduite (20 mm de mercure; 2,7 kPa) à une température voisine de 20° C en présence de potasse en pastilles. On obtient ainsi 10,55 g de [(chloro-2 phényl)-4 pipérazinyl-1]-5 pyrrolidinone-2 sous forme de cristaux blancs fondant à 198° C.

*Exemple 7:*

Un mélange de 7,75 g d'étoxy-5 pyrrolidinone-2 et de 11,55 g de (méthoxy-4 phényl)-1 pipérazine est chauffé sous agitation pendant 1 h et 10 min à une température voisine de 140° C en

distillant l'éthanol formé. Après refroidissement du mélange réactionnel, le solide obtenu est séparé par filtration lavé 3 fois par 20 $cm^3$ au total d'acétate d'éthyle et séché sous pression réduite (20 mm de mercure; 2,7 kPa) à une température voisine de 20° C en présence de potasse en pastilles. On obtient ainsi 12,5 g de produit brut fondant à 184° C. Ce produit est dissous dans 245 $cm^3$ d'acétonitrile bouillant; la solution est additionnée de 1 g de noir décolorant et filtrée à chaud. Le filtrat est refroidi à une température voisine de 4° C pendant 16 h. Les cristaux apparus sont séparés par filtration, lavés 2 fois par 20 $cm^3$ au total d'acétonitrile et séchés sous pression réduite (20 mm de mercure; 2,7 kPa) à une température voisine de 20° C en présence de potasse en pastilles. On obtient ainsi 10,9 g de [(méthoxy-4 phényl)-4 pipérazinyl-1]-5 pyrrolidinone-2 sous forme de cristaux blancs fondant à 189° C.

*Exemple 8:*

Un mélange de 7,75 g d'étoxy-5 pyrrolidinone-2 et de 12,26 g d'(acétyl-4 phényl)-1 pipérazine est chauffé sous agitation pendant 1 h et 40 min à une température comprise entre 126 et 140° C en distillant l'éthanol formé. Après refroidissement du mélange réactionnel, le solide obtenu est séparé par filtration, lavé 3 fois par 20 $cm^3$ au total d'acétate d'éthyle et séché sous pression réduite (20 mm de mercure; 2,7 kPa) à une température voisine de 20° C en présence de potasse en pastilles. On obtient ainsi 15,6 g de produit brut fondant à 210° C. Ce produit est dissous dans 80 $cm^3$ de butanol bouillant; la solution est additionnée de 1 g de noir décolorant et filtrée à chaud. Le filtrat est refroidi à une température voisine de 4° C pendant 16 h. Les cristaux apparus sont séparés par filtration, lavés 2 fois par 20 $cm^3$ au total de butanol refroidi à une température voisine de 4° C puis 3 fois par 60 $cm^3$ au total d'oxyde d'isopropyle et séchés sous pression réduite (20 mm de mercure; 2,7 kPa) à une température voisine de 20° C en présence de potasse en pastilles. On obtient ainsi 12,1 g de produit fondant à 210° C. 11,5 g de ce produit sont repris par 80 $cm^3$ d'une solution aqueuse 0,5N d'acide chlorhydrique. La solution est additionnée de 1 g de noir décolorant et filtrée; le filtrat est refroidi à une température voisine de 4° C et alcalinisé par 50 $cm^3$ d'une solution aqueuse normale de soude. Après 10 min à une température voisine de 4° C, les cristaux apparus sont séparés par filtration, lavés 3 fois par 30 $cm^3$ au total d'eau distillée refroidie à une température voisine de 4° C et séchés sous pression réduite (20 mm de mercure; 2,7 kPa) à une température voisine de 20° C en présence de potasse en pastilles. On obtient ainsi 9,7 g de produit fondant à 180° C. Ce produit est dissous dans 80 $cm^3$ de butanol bouillant et la solution obtenue est additionnée de 1 g de noir décolorant et filtrée à chaud. Le filtrat est refroidi à une température voisine de 4° C pendant 1 h. Les cristaux obtenus sont séparés par filtration, lavés 2 fois par 20 $cm^3$ au total de butanol et séchés sous pression réduite (20 mm de mercure; 2,7 kPa) à une température voisine de 20° C en présence de potasse en pastilles. On obtient ainsi 6,7 g d'[(acétyl-4 phényl)-4 pipérazinyl-1]-5 pyrrolidinone-2 sous forme de cristaux jaunes fondant à 199° C.

*Exemple 9:*

Un mélange de 7,75 g d'étoxy-5 pyrrolidinone-2 et de 11,1 g de benzyl-4 pipérazine est chauffé sous agitation pendant 1 h et 10 min à une température comprise entre 140 et 145° C en distillant l'éthanol formé. Après refroidissement du mélange réactionnel, le solide obtenu est mis en suspension dans 40 $cm^3$ d'acétate d'éthyle, séparé par filtration, lavé 3 fois par 25 $cm^3$ au total d'acétate d'éthyle et séché sous pression réduite (20 mm de mercure; 2,7 kPa) à une température voisine de 20° C en présence de potasse en pastilles. On obtient ainsi 11,9 g de produit brut fondant à 159° C. Ce produit est dissous dans 210 $cm^3$ d'acétate d'éthyle bouillant; la solution est additionnée de 1 g de noir décolorant et filtrée à chaud. Le filtrat est refroidi à une température voisine de 4° C pendant 16 h. Les cristaux apparus sont séparés par filtration, lavé 3 fois par 30 $cm^3$ au total d'acétate d'éthyle refroidi à une température voisine de 4° C et séchés sous pression réduite (20 mm de mercure; 2,7 kPa) à une température voisine de 20° C en présence de potasse en pastilles. On obtient ainsi 9,78 g de (benzyl-4 pipérazinyl-1)-5 pyrrolidinone-2 sous forme de cristaux blancs fondant à 160° C.

*Exemple 10:*

Un mélange de 12,9 g d'étoxy-5 pyrrolidinone-2 et de 24,2 g de (trifluorométhyl-3 phényl)-1 pipérazine est chauffé sous agitation pendant 35 min à une température comprise entre 155 et 164° C en distillant l'éthanol formé. Après reroidissement du mélange réactionnel, le solide obtenu est séparé par filtration, lavé 4 fois par 85 $cm^3$ au total d'oxyde d'isopropyle et séché sous pression réduite (20 mm de mercure; 2,7 kPa) à une température voisine de 20° C en présence de potasse en pastilles. On obtient ainsi 24,4 g de produit brut fondant à 148-150° C. Ce produit est dissous dans 120 $cm^3$ de méthanol bouillant; la solution est additionnée de 1 g de noir décolorant et filtrée à chaud. Le filtrat est refroidi à une température voisine de 4° C pendant 16 h. Les cristaux apparus sont séparés par filtration, lavés 2 fois par 20 $cm^3$ au total de méthanol et séchés sous pression réduite (20 mm de mercure; 2,7 kPa) à une température voisine de 20° C en présence de potase en pastilles. On obtient ainsi 18,9 g de [(trifluorométhyl-3 phényl)-4 pipérazinyl-1]-5 pyrrolidinone-2 sous forme de cristaux blancs fondant à 166° C.

*Exemple 11:*

Un mélange de 6,45 g d'étoxy-5 pyrrolidinone-2 et de 10 g de (trifluorométhyl-4 phényl)-1 pipérazine est chauffé sous agitation à une température voisine de 145° C pendant 1 h en distillant l'éthanol formé. Après refroidissement du mélange réactionnel, on ajoute 10 $cm^3$ d'éther diéthylique pour mettre en suspension le solide obtenu. Les cristaux

sont ensuite séparés par filtration, lavés 3 fois par 25 $cm^3$ au total d'éther diéthylique et séchés à l'air. On obtient ainsi 10,45 g de produit brut fondant à 188° C. Ce produit est dissous dans 100 $cm^3$ d'éthanol bouillant; la soution obtenue est additionnée de 0,5 g de noir décolorant et filtrée à chaud. Le filtrat est refroidi à une température voisine de 4° C pendant 1 h. Les cristaux apparus sont séparés par filtration, lavés 3 fois par 20 $cm^3$ au total d'éthanol refroidi à une température voisine de 4° C et séchés sous pression réduite (20 mm de mercure; 2,7 kPa) à une température voisine de 20° C en présence de potasse en pastilles. On obtient ainsi 8,9 g de [(trifluorométhyl-4 phényl)-4 pipérazinyl-1]-5 pyrrolidinone-2 sous forme de cristaux blancs fondant à 189° C.

*Exemple 12:*

Un mélange de 8,5 g d'étoxy-5 pyrrolidinone-2 et de 10,6 g de (méthyl-4 phényl)-1 pipérazine est chauffé sous agitation pendant 1 h et 20 min à une température voisine de 140° C en distillant l'éthanol formé. Après refroidissement du mélange réactionnel, on ajoute 20 $cm^3$ d'éther diéthylique pour mettre en suspension le solide obtenu. Les cristaux sont ensuite séparés par filtration, lavés 3 fois par 60 $cm^3$ au total d'éther diéthylique et séchés sous pression réduite (20 mm de mercure; 2,7 kPa) à une température voisine de 20° C en présence de potasse en pastilles. On obtient ainsi 11,3 g de produit fondant à 202° C. Ce produit est dissous dans 340 $cm^3$ d'éthanol bouillant. La solution obtenue est additionnée de 0,5 g de noir décolorant et filtrée à chaud. Le filtrat est refroidi à une température voisine de 4° C pendant 16 h. Les cristaux apparus sont séparés par filtration, lavés 2 fois par 20 $cm^3$ au total d'éthanol et 2 fois par 50 $cm^3$ au total d'éther diéthylique puis séchés sous pression réduite (20 mm de mercure; 2,7 kPa) à une température voisine de 20° C en présence de potasse en pastilles. On obtient ainsi 10,4 g de [(méthyl-4 phényl)-4 pipérazinyl-1]-5 pyrrolidinone-2 sous forme de cristaux blancs fondant à 204° C.

*Exemple 13:*

Un mélange de 7,15 g d'étoxy-5 pyrrolidinone-2 et 9,75 g de (méthyl-3 phényl)-1 pipérazine est chauffé sous agitation pendant 2 h à une température voisine de 145° C en distillant l'éthanol formé. Après refroidissement du mélange réactionnel, on ajoute 210 $cm^3$ d'acétonitrile et porte à reflux pour dissoudre le solide obtenu. La solution est additionnée de 0,5 g de noir décolorant et filtrée à chaud. Le filtrat est refroidi à une température voisine de 4° C pendant 1 h. Les cristaux apparus sont séparés par filtration, lavés 3 fois par 25 $cm^3$ au total d'acétonitrile refroidi à une température voisine de 4° C puis 3 fois par 45 $cm^3$ au total d'oxyde d'isopropyle et ensuite séchés sous pression réduite (20 mm de mercure; 2,7 kPa) à une température voisine de 20° C en présence de potasse en pastilles. On obtient ainsi 9,8 g de [(méthyl-3 phényl)-4 pipérazinyl-1]-5 pyrrolidinone-2 sous forme de cristaux crème fondant à 148° C.

*Exemple 14:*

Un mélange de 7,1 g d'étoxy-5 pyrrolidinone-2 et de 10 g de (nitro-4 phényl)-1 pipérazine est chauffé sous agitation pendant 1 h et 40 min à une température comprise entre 130 et 143° C en distillant l'éthanol formé. Après refroidissement, on filtre le mélange réactionnel et le solide obtenu est lavé 4 fois par 55 $cm^3$ au total d'éther diéthylique et séché sous pression réduite (20 mm de mercure; 2,7 kPa) à une température voisine de 20° C en présence de potasse en pastilles. On obtient ainsi 13,9 g de produit brut. Ce produit est dissous dans 500 $cm^3$ d'acétonitrile bouillant; la solution est additionnée de 0,5 g de noir décolorant et filtrée à chaud. Le filtrat est refroidi à une température voisine de 4° C pendant 16 h. Les cristaux apparus sont séparés par filtration, lavés 2 fois par 20 $cm^3$ au total d'acétonitrile et 1 fois par 25 $cm^3$ d'éther diéthylique puis séchés sous pression réduite (20 mm de mercure; 2,7 kPa) à une température voisine de 20° C en présence de potasse en pastilles. On obtient ainsi 10,4 g de [(nitro-4 phényl)-4 pipérazinyl-1]-5 pyrrolidinone-2 sous forme de cristaux jaunes fondant à 200° C.

*Exemple 15:*

Un mélange de 7,1 g d'étoxy-5 pyrrolidinone-2 et de 9,25 g de (méthoxy-3 phényl)-1 pipérazine est chauffé sous agitation pendant 1 h et 20 min à une température comprise entre 132 et 148° C en distillant l'éthanol formé. Après refroidissement du mélange réactionnel, on ajoute 25 $cm^3$ d'éther diéthylique pour mettre en suspension le solide obtenu. Les cristaux sont ensuite séparés par filtration, lavés 3 fois par 45 $cm^3$ au total d'éther diéthylique et séchés sous pression réduite (20 mm de mercure; 2,7 kPa) à une température voisine de 20° C en présence de potasse en pastilles. On obtient ainsi 12,2 g de produit brut fondant à 140° C. Ce produit est dissous dans 150 $cm^3$ d'éthanol bouillant. La solution obtenue est additionnée de 0,5 g de noir décolorant et filtrée à chaud. Le filtrat est refroidi à une température voisine de 4° C pendant 4 h. Les cristaux apparus sont séparés par filtration, lavés 3 fois par 30 $cm^3$ au total d'éthanol et séchés sous pression réduite (20 mm de mercure; 2,7 kPa) à une température voisine de 20° C en présence de potasse en pastilles. On obtient ainsi 9,45 g de [(méthoxy-3 phényl)-4 pipérazinyl-1]-5 pyrrolidinone-2 sous forme de cristaux crème fondant à 156° C.

*Exemple 16:*

Un mélange de 8,3 g d'étoxy-5 pyrrolidinone-2 et de 9,9 g d'(hydroxy-4 phényl)-1 pipérazine est chauffé sous agitation à une température voisine de 127° C en distillant l'éthanol formé. 15 min après le début du chauffage, on observe une prise en masse; on ajoute alors 40 $cm^3$ de xylène pour déliter le mélange réactionnel et on poursuit le chauffage pendant 35 min à une température comprise entre 135 et 140° C. La suspension obtenue est refroidie à une température voisine de 40° C; le solide est séparé par filtration, lavé 1 fois par

10 $cm^3$ de xylène et 3 fois par 30 $cm^3$ au total d'acétate d'éthyle, puis séché sous pression réduite (20 mm de mercure; 2,7 kPa) à une température voisine de 20° C en présence de potasse en pastilles. On obtient ainsi 13,95 g de produit brut. Ce produit est dissous dans 155 $cm^3$ de diméthylformamide à une température voisine de 110° C. La solution obtenue est additionnée de 0,5 g de noir décolorant et filtrée à chaud. Le filtrat est refroidi à une température voisine de 4° C pendant 3 h. Les cristaux apparus sont séparés par filtration, lavés 3 fois par 30 $cm^3$ au total de diméthylformamide et 3 fois par 150 $cm^3$ au total d'acétate d'éthyle et séchés sous pression réduite (20 mm de mercure; 2,7 kPa) à une température voisine de 20° C en présence de potasse en pastilles. On obtient ainsi 9,2 g d'[(hydroxy-4 phényl)-4 pipérazinyl-1]-5 pyrrolidinone-2 sous forme de cristaux crème fondant à 270° C.

*Exemple 17:*

Un mélange de 5,2 g d'étoxy-5 pyrrolidinone-2 et de 8,1 g de (dichloro-3,4 phényl)-1 pipérazine est chauffé sous agitation pendant 1 h et 30 min à une température comprise entre 100 et 140° C en distillant l'éthanol formé. Après refroidissement du mélange réactionnel, on ajoute 20 $cm^3$ d'éther et 40 $cm^3$ d'éthanol. Les cristaux qui apparaissent alors sont séparés par filtration. Après addition de 40 $cm^3$ d'éthanol au filtrat, des cristaux apparaissent à nouveau; ils sont séparés par filtration, réunis à ceux obtenus précédemment, lavés 3 fois par 45 $cm^3$ au total d'éther diéthylique et séchés sous pression réduite (20 mm de mercure; 2,7 kPa) à une température voisine de 20° C en présence de potasse en pastilles. On obtient ainsi 8,4 g de produit brut. Ce produit est dissous dans 170 $cm^3$ d'éthanol bouillant. La solution obtenue est additionnée de 0,5 g de noir décolorant et filtrée à chaud. Le filtrat est refroidi à une température voisine de 4° C pendant 3 h. Les cristaux apparus sont séparés par filtration, lavés 3 fois par 30 $cm^3$ au total d'éthanol et séchés sous pression réduite (20 mm de mercure; 2,7 kPa) à une température voisine de 20° C en présence de potasse en pastilles. On obtient ainsi 6,5 g de [(dichloro-3,4 phényl)-4 pipérazinyl-1]-5 pyrrolidinone-2 sous forme de cristaux blancs fondant à 186° C.

La (dichloro-3,4 phényl)-1 pipérazine peut être préparée comme décrit par L. Thunus, C.L. Lapiere et N. Verbeke [«Ann. Pharm. Fr.», *38*, 353, (1980)].

*Exemple 18:*

Un mélange de 3,2 g d'étoxy-5 pyrrolidinone-2 et de 4,4 g de (diméthylamino-4 phényl)-1 pipérazine est chauffé sous agitation pendant 35 min à une température voisine de 145° C. Après refroidissement du mélange réactionnel, le solide obtenu est séparé par filtration, lavé 3 fois par 60 $cm^3$ au total d'éther diéthylique et séché à l'air. On obtient ainsi 4,5 g de produit brut fondant à 210° C. Ce produit est dissous dans 155 $cm^3$ d'éthanol bouillant. La solution obtenue est additionnée de 0,5 g de noir décolorant et filtrée à chaud. Le filtrat est refroidi à une température voisine de 20° C pendant 3 h. Les cristaux apparus sont séparés par filtration, lavés 2 fois par 10 $cm^3$ au total d'éthanol et 2 fois par 10 $cm^3$ au total d'éther diéthylique et séchés sous pression réduite (20 mm de mercure; 2,7 kPa) à une température voisine de 20° C en présence de potasse en pastilles. On obtient ainsi 2,9 g de [(diméthylamino-4 phényl)-4 pipérazinyl-1]-5 pyrrolidinone-2 sous forme de cristaux crème fondant à 210° C.

La (diméthylamino-4 phényl)-1 pipérazine peut être préparée de la façon suivante:

Un mélange de 25 g de dichlorhydrate de N,N-diméthyl paraphénylènediamine, de 21,4 g de chlorhydrate de bis(chloro-2 éthyl)amine et de 33,2 g de carbonate de potassium dans 80 $cm^3$ de butoxy-2 éthanol est chauffé sous agitation pendant 17 h à une température voisine de 155° C. Après refroidissement du mélange réactionnel, on ajoute 400 $cm^3$ d'eau distillée et lave trois fois la phase aqueuse avec 450 $cm^3$ au total d'actétate d'éthyle. La phase aqueuse est amenée à un pH voisin de 10 par addition d'une solution aqueuse de soude 10N, saturée par du chlorure de sodium puis extraite à nouveau 3 fois par 450 $cm^3$ au total d'acétate d'éthyle. Les extraits organiques sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 40° C. On obtient ainsi 21 g de produit. Ce produit est dissous dans 210 $cm^3$ d'éthanol. La solution obtenue est additionnée de 35 $cm^3$ d'une solution éthanolique d'acide chlorhydrique 7N et est refroidie à une température voisine de 4° C pendant 2 h. Les cristaux apparus sont séparés par filtration, lavés 3 fois par 75 $cm^3$ au total d'éthanol et 2 fois par 100 $cm^3$ au total d'éther diéthylique puis séchés sous pression réduite (20 mm de mercure; 2,7 kPa) à une température voisine de 20° C en présence de potasse en pastilles. On obtient ainsi 18 g de produit fondant à 200-205° C. Ce produit est dissous dans 180 $cm^3$ d'eau distillée. La solution obtenue est amenée à un pH voisin de 11 par addition de 13 $cm^3$ d'une solution de soude 10N, saturée par du chlorure de sodium, extraite 4 fois par 400 $cm^3$ au total d'acétate d'éthyle. Les extraits organiques sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 40° C. On obtient ainsi 7,2 g de produit. Ce produit est chromatographié sur une colonne de 4 cm de diamètre contenant 73 g de silice (0,063-0,2 mm) en éluant avec du méthanol et en recueillant des fractions de 100 $cm^3$. La première fraction est éliminée; les 7 fractions suivantes sont réunies et concentrées à sec sous pression réduite (20 mm de mercure; 2,7 kPa) à une température voisine de 45° C. On obtient ainsi 4,5 g de (diméthylamino-4 phényl)-1 pipérazine sous la forme d'une huile brune qui cristallise (Rf = 0,15; chromatographie sur couche mince de gel de silice; éluant: méthanol).

*Exemple 19:*

Un mélange de 4,5 g d'étoxy-5 pyrrolidinone-2 et de 9,3 g de (chloro-4 trifluorométhyl-3 phényl)-4 pipérazine est chauffé sous agitation pendant 1 h à une température comprise entre 130 et 139° C en distillant l'éthanol formé. Après refroidissement, le mélange réactionnel est dilué par 25 $cm^3$ d'éthanol et la solution obtenue est agitée à une température voisine de 20° C pendant 16 h. Un produit précipite; on ajoute alors au mélange réactionnel 25 $cm^3$ d'éther diéthylique; les cristaux sont séparés par filtration, lavés 3 fois par 60 $cm^3$ au total d'éther diéthylique et séchés sous pression réduite (20 mm de mercure; 2,7 kPa) à une température voisine de 20° C en présence de potasse en pastilles. On obtient ainsi 6,2 g de produit brut fondant à 152° C. Ce produit est dissous dans 60 $cm^3$ d'acétate d'éthyle bouillant; la solution est additionnée de 1 g de noir décolorant et filtrée à chaud. Le filtrat est refroidi à une température voisine de 4° C pendant 3 h. Les cristaux apparus sont séparés par filtration, lavés 2 fois par 15 $cm^3$ au total d'acétate d'éthyle et séchés sous pression réduite (20 mm de mercure; 2,7 kPa) à une température voisine de 20° C en présence de potasse en pastilles. On obtient ainsi 4,8 g de [(chloro-4 trifluorométhyl-3 phényl)-4 pipérazinyl-1]-5 pyrrolidinone-2 sous forme de cristaux blancs fondant à 155° C.

*Exemple 20:*

Un mélange de 4,7 g d'étoxy-5 pyrrolidinone-2 et de 8,3 g de (triméthoxy-3,4,5 phényl)-4 pipérazine est chauffé sous agitation pendant 1 h à une température voisine de 140° C en distillant l'éthanol formé. Après refroidissement du mélange réactionnel, on ajoute 20 $cm^3$ d'éthanol pour mettre en suspension le solide obtenu. Les cristaux sont ensuite séparés par filtration, lavés 3 fois par 6 $cm^3$ au total d'éthanol, et séchés sous pression réduite (20 mm de mercure; 2,7 kPa) à une température voisine de 20° C en présence de potasse en pastilles. On obtient ainsi 3,1 g de produit brut fondant à 138° C. Les liqueurs mères de cristallisation sont concentrées à sec sous pression réduite (20 mm de mercure; 2,7 kPa) à une température voisine de 50° C. On obtient ainsi 9,5 g de produit que l'on chromatographie sur une colonne de 3 cm de diamètre contenant 240 g de silice (0,04-0,063 mm). On élue avec un mélange d'acétate d'éthyle et de méthanol (80/20 en volumes) sous une pression de 0,5 bar (51 kPa) en recueillant des fractions de 50 $cm^3$. Les 10 premières fractions sont éliminées, les 12 suivantes sont réunies, concentrées à sec sous pression réduite (20 mm de mercure; 2,7 kPa) à une température voisine de 50° C. On obtient ainsi 3,4 g de produit fondant à 130° C. On réunit ce produit aux 3,1 g de produit obtenus précédemment et on ajoute 80 $cm^3$ d'acétate d'éthyle bouillant. La solution obtenue est additionnée de 0,5 g de noir décolorant et filtrée à chaud; le filtrat est refroidi à une température voisine de 4° C pendant 1 h. Les cristaux apparus sont séparés par filtration, lavés 3 fois par 20 $cm^3$ au total d'acétate d'éthyle refroidi à une température voisine de 4° C et séchés sous pression réduite (20 mm de mercure; 2,7 kPa) à une température voisine de 20° C en présence de potasse en pastilles. On obtient ainsi 4,5 g de [(triméthoxy-3,4,5 phényl)-4 pipérazinyl-1]-5 pyrrolidinone-2 sous forme de cristaux crème fondant à 140° C.

*Exemple 21:*

Un mélange de 8,5 g d'étoxy-5 pyrrolidinone-2 et de 9,1 g de (nitro-2 phényl)-1 pipérazine est chauffé sous agitation pendant 1 h et 30 min à une température voisine de 140° C en distillant l'éthanol formé. Après refroidissement du mélange réactionnel, on ajoute 20 $cm^3$ d'éther diéthylique pour mettre en suspension le solide obtenu. Les cristaux sont ensuite séparés par filtration, lavés 3 fois par 60 $cm^3$ au total d'éther diéthylique et séchés à l'air. On obtient ainsi 12 g de produit brut fondant à 196° C. Ce produit est dissous dans 575 $cm^3$ d'éthanol bouillant. La solution obtenue est additionnée de 0,5 g de noir décolorant et filtrée à chaud. Le filtrat est refroidi à une température voisine de 4° C pendant 48 h. Les cristaux apparus sont séparés par filtration, lavés 3 fois par 45 $cm^3$ au total d'éthanol et séchés sous pression réduite (20 mm de mercure; 2,7 kPa) à une température voisine de 20° C en présence de potasse en pastilles. On obtient ainsi 10,3 g de [(nitro-2 phényl)-4 pipérazinyl-1]-5 pyrrolidinone-2 sous forme de cirstaux orangés fondant à 197° C.

*Exemple 22:*

A une solution de 14,5 g de phényl-3 pyrrolidinedione-2,5 dans 580 $cm^3$ d'éthanol, on ajoute 4,7 g de borohydrure de sodium; la suspension obtenue est refroidie aussitôt à une température comprise entre 0 et −5° C. On ajoute alors en 2 h à une température comprise entre 0 et −5° C 11,7 $cm^3$ d'une solution éthanolique d'acide chlorhydrique 7,1 N. La suspension est agitée à une température voisine de 0° C pendant 2 h et 30 min. On ajoute à nouveau 6 $cm^3$ d'une solution éthanolique d'acide chlorhydrique 7,1 N et poursuit l'agitation à une température voisine de 0° C pendant 1 h. On ajoute alors 50 $cm^3$ d'une solution éthanolique de potasse à 10% (poids/volume) et agite le mélange réactionnel à une température voisine de 20° C pendant 16 h. Le solide obtenu est séparé par filtration et le filtrat est concentré à sec sous pression réduite (20 mm de mercure; 2,7 kPa) à une température voisine de 45° C. Le produit obtenu (19,7 g) est repris par 190 $cm^3$ d'éther diéthylique et l'insoluble est séparé par filtration. Le filtrat est concentré à sec sous pression réduite (20 mm de mercure; 2,7 kPa) à une température voisine de 50° C. On obtient ainsi 14 g de produit. A ce produit, on ajoute 11 g de N-phénylpipérazine et la solution obtenue est chauffée à une température voisine de 140° C pendant 45 min. Après refroidissement du mélange réactionnel, la solution obtenue est diluée par 50 $cm^3$ de méthanol puis agitée à une température voisine de 20° C pendant 3 h. Les cristaux apparus sont séparés par filtration, lavés 2 fois par 20 $cm^3$ au

total de méthanol et 2 fois par 20 $cm^3$ au total d'éther diéthylique puis séchés sous pression réduite (20 mm de mercure; 2,7 kPa) à une température voisine de 20° C en présence de potasse en pastilles. On obtient ainsi 9,6 g de produit fondant à 158-160° C. Ce produit est dissous dans 290 $cm^3$ d'éthanol bouillant; la solution obtenue est additionnée de 0,5 g de noir décolorant et filtrée à chaud. Le filtrat est refroidi à une température voisine de 4° C pendant 48 h. Les cristaux apparus sont séparés par filtration, lavés 2 fois par 20 $cm^3$ au total d'éthanol et 2 fois par 20 $cm^3$ au total d'éther diéthylique puis séchés sous pression réduite (20 mm de mercure; 2,7 kPa) à une température voisine de 20° C en présence de potasse en pastilles. On obtient ainsi 7,3 g de phényl-3 (phényl-4 pipérazinyl-1)-5 pyrrolidinone-2 sous forme de cristaux blancs fondant à 174° C.

La phényl-3 pyrrolidinedione-2,5 peut être préparée comme décrit par R. Wegscheider et J. Hecht, «Monatsh.», *24*, 422 (1903).

*Exemple 23:*

Un mélange de 9 g d'étoxy-5 pyrrolidinone-2 et de 8 g de phényl-4 pipéridine est chauffé sous agitation pendant 20 min à une température voisine de 140° C en distillant l'éthanol formé. Après refroidissement du mélange réactionnel, le solide obtenu est séparé par filtration, lavé 3 fois par 25 $cm^3$ au total d'acétate d'éthyle et séché sous pression réduite (20 mm de mercure; 2,7 kPa) à une température voisine de 20° C en présence de potasse en pastilles. On obtient ainsi 10,7 g de produit brut fondant à 198° C. Ce produit est dissous dans 430 $cm^3$ d'acétonitrile bouillant. La solution obtenue est additionnée de 0,5 g de noir décolorant et filtrée à chaud. Le filtrat est refroidi à une température voisine de 4° C pendant 4 h. Les cristaux apparus sont séparés par filtration, lavés 3 fois par 30 $cm^3$ au total d'acétonitrile et séchés sous pression réduite (20 mm de mercure; 2,7 kPa) à une température voisine de 20° C en présence de potasse en pastilles. On obtient ainsi 8,6 g de (phényl-4 pipéridino)-5 pyrrolidinone-2 sous forme de cristaux blancs fondant à 200° C.

*Exemple 24:*

Une suspension de 19,6 g de (phényl-4 pipérazinyl-1)-5 pyrrolidinone-2 dans 300 $cm^3$ de diméthylformamide est ajoutée en 50 min, à une température comprise entre 22 et 28° C, à une suspension de 4,6 g d'hydrure de sodium (en dispersion à 50% dans l'huile de vaseline) dans 50 $cm^3$ de diméthylformamide. Le mélange réactionnel est maintenu sous agitation pendant 2 h à une température voisine de 23° C. On ajoute ensuite en 15 min, à une température comprise entre 23 et 33° C, une solution de 12,4 g d'iodure de méthyle dans 50 $cm^3$ de diméthylformamide. Après 16 h d'agitation supplémentaire à une température voisine de 20° C, le milieu réactionnel est filtré et l'insoluble est lavé 2 fois par 100 $cm^3$ au total de diméthylformamide. Les filtrats sont réunis, concentrés à sec sous pression réduite (5 mm de mercure; 0,7 kPa) à une température voisine de 40° C. Le produit obtenu est dissous dans 200 $cm^3$ d'eau distillée à une température voisine de 50° C. Après refroidissement à une température voisine de 20° C, les cristaux apparus sont séparés par filtration, lavés 2 fois par 50 $cm^3$ au total d'eau distillée refroidie à une température voisine de 4° C puis séchés à l'air à une température voisine de 20° C. On obtient ainsi 21 g de produit brut hydraté fondant à une température voisine de 50° C. Ce produit est dissous dans 500 $cm^3$ d'oxyde d'isopropyle bouillant. L'eau apparue est séparée par décantation et la phase organique est filtrée à chaud. Après refroidissement à une température voisine de 4° C pendant 2 h, les cristaux apparus sont séparés par filtration, lavés 3 fois par 150 $cm^3$ au total d'oxyde d'isopropyle et séchés sous pression réduite (20 mm de mercure; 2,7 kPa) à une température voisine de 20° C en présence de potasse en pastilles puis sous 1 mm de mercure (0,14 kPa) à 20° C jusqu'à poids constant. On obtient ainsi 15,3 g de produit hydraté et hygroscopique. Laissé à l'air jusqu'à poids constant, ce produit absorbe 500 mg d'eau. On obtient ainsi finalement le monohydrate de la méthyl-1 (phényl-4 pipérazinyl-1)-5 pyrrolidinone-2 sous forme de cristaux blancs fondant à 84° C.

La (phényl-4 pipérazinyl-1)-5 pyrrolidinone-2 est préparée comme à l'exemple 1.

*Exemple 25:*

Une suspension de 52 g de (phényl-4 pipérazinyl-1)-5 pyrrolidinone-2 et de 53,5 g de bis(méthoxy-4 phényl)-2,4 dithioxo-2,4 dithia-1,3 diphospha-2,4 étanne dans 1000 $cm^3$ de diméthoxy-1,2 éthane est agitée à une température voisine de 20° C pendant 24 h. Les cristaux apparus sont séparés par filtration, lavés 2 fois par 100 $cm^3$ au total de diméthoxy-1,2 éthane et 3 fois par 300 $cm^3$ au total d'oxyde d'isopropyle et séchés sous pression réduite (20 mm de mercure; 2,7 kPa) à une température voisine de 20° C en présence de potasse en pastilles. On obtient ainsi 8,4 g de produit brut fondant à 200° C. 8 g de ce produit sont réunis à 0,3 g de produit préparé de la même manière dans une opération antérieure et sont dissous dans 800 $cm^3$ d'éthanol bouillant. On ajoute 0,5 g de noir décolorant et filtre à chaud; le filtrat est refroidi à une température voisine de 4° C pendant 1 h. Les cristaux apparus sont séparés par filtration, lavés 3 fois par 150 $cm^3$ au total d'éthanol puis 3 fois par 150 $cm^3$ au total d'éther diéthylique et séchés sous pression réduite (20 mm de mercure; 2,7 kPa) à une température voisine de 20° C en présence de potasse en pastilles. Les 6,3 g de produit ainsi obtenus sont dissous dans 120 $cm^3$ de butanol-1 bouillant. Après refroidissement à une température voisine de 20° C pendant 1 h, les cristaux apparus sont séparés par filtration, lavés 2 fois par 20 $cm^3$ au total de butanol-1 puis 3 fois par 75 $cm^3$ au total d'éthanol et enfin 3 fois par 75 $cm^3$ au total d'éther diéthylique et séchés sous pression réduite (20 mm de mercure; 2,7 kPa) à une température voisine de 20° C en présence de potasse en pastilles. On obtient ainsi 5,6 g de (phényl-4 pipérazinyl-1)-5 pyrrolidine-

thione-2 sous forme de cristaux blancs fondant à 210° C.

La (phényl-4 pipérazinyl-1)-5 pyrrolidinone-2 est préparée comme indiqué à l'exemple 1.

La présente invention concerne également les médicaments constitués par un produit de formule générale (I), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que: amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions sériles pour administration parentérale peuvent étre de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi stérile injectable.

Les compositions pour administration rectale sont les suppositoires ou les capsules rectales, qui contiennent outre le produit actif des excipients tels que le beurre de cacao, des glycérides semisynthétiques ou des polyéthylèneglycols.

En thérapeutique humaine, les produits selon l'invention sont particulièrement utiles dans le traitement des syndromes des états dépressifs divers et des états psychasthéniques. Les doses dépendent de l'effet recherché et de la durée du traitement; elles sont généralement comprises entre 10 et 300 mg par jour par voie orale ou sous-cutanée pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

*Exemple A:*

On prépare, selon la technique habituelle, des comprimés dosés à 10 mg de produit actif ayant la composition suivante:

| | |
|---|---|
| (phényl-4 pipérazinyl-1)-5 pyrrolidine thione-2 | 10 mg |
| amidon | 60 mg |
| lactose | 50 mg |
| stéarate de magnésium | 2 mg |

*Exemple B:*

On prépare des ampoules contenant chacune 10 mg de produit actif en préparant une solution contenant:

| | |
|---|---|
| [(méthoxy-4 phényl)-4 pipérazinyl-1]-5 pyrrolidinone-2 | 100 mg |
| solution aqueuse d'acide chlorhydrique 0,1 N | 3,65 $cm^3$ |
| eau distillée q.s.p. | 20 $cm^3$ |

puis en répartissant la solution obtenue de façon égale dans 10 ampoules.

**Revendications** pour les Etats contractants BE, CH, DE, FR, GB, IT, LI, LU, NL, SE

1. Dérivé de la pyrrolidine, caractérisé en ce qu'il répond à la formule générale:

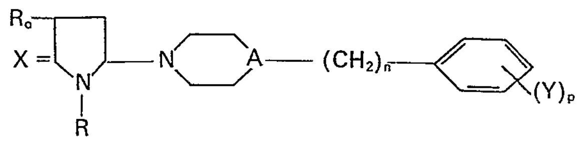

dans laquelle X représente un atome d'oxygène ou de soufre, R représente un atome d'hydrogène ou un radical alcoyle, $R_o$ représente un atome d'hydrogène ou un radical alcoyle ou phényle éventuellement substitué par un atome d'halogène ou un radical alcoyle, alcoyloxy, alcoylthio ou nitro, A représente un atome d'azote ou un radical =CH–, les symboles Y, identiques ou différents, représentent un atome d'hydrogène ou d'halogène ou un radical alcoyle, alcoyloxy, alcoylthio, alcoylcarbonyle, nitro, amino, alcoylcarbonylamino, alcoylamino, dialcoylamino, alcoylsulfinyle, alcoylsulfonyle, alcoyloxycarbonyle, cyano, trifluorométhyle, hydroxy, mercapto, alcoylcarbonyloxy ou alcoylcarbonylthio et n représente le nombre 0 ou 1 et p représente le nombre 1, 2 ou 3, étant entendu que tous les radicaux alcoyle ou portions alcoyle sont en chaîne droite ou ramifiée et contiennent 1 à 4 atomes de carbone, ainsi que leurs sels d'addition avec des acides.

2. Procédé pour la préparation d'un produit selon la revendication 1 dans la formule duquel X représente un atome d'oxygène et les autres symboles sont définis comme à la revendication 1, caractérisé en ce que l'on fait réagir un produit de formule générale:

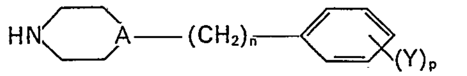

dans laquelle A, Y, n et p sont définis comme à la revendication 1 sur une pyrrolidinone-2 de formule générale:

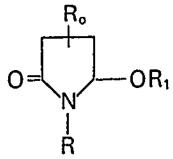

dans laquelle R et $R_o$ sont définis comme à la revendication 1 et $R_1$ représente un radical alcoyle, isole le produit attendu et le transforme éventuellement en un sel d'addition avec un acide.

3. Procédé pour la préparation d'un produit selon la revendication 1 dans la formule duquel $R_o$, A, Y, n et p sont définis comme à la revendication 1, X représente un atome d'oxygène et R représente un radical alcoyle, caractérisé en ce qu'on fait réagir un dérivé de formule générale:

R'Z

dans laquelle R' représente un radical alcoyle et Z représente un atome d'halogène ou un reste d'ester réactif sur un produit de formule générale:

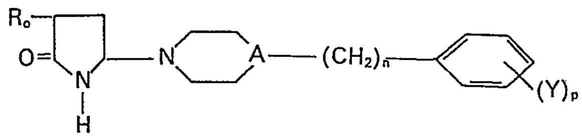

dans laquelle $R_o$, A, Y, n et p sont définis comme à la revendication 1, puis isole le produit obtenu et le transforme éventuellement en un sel d'addition avec un acide.

4. Procédé pour la préparation d'un produit selon la revendication 1 dans la formule duquel X représente un atome de soufre et les autres symboles sont définis comme à la revendication 1, caractérisé en ce que l'on transforme par thionation un composé selon la revendication 1 dans la formule duquel X représente un atome d'oxygène, c'est-à-dire un composé de formule générale:

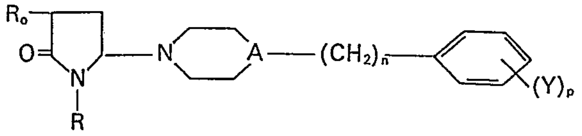

puis isole le produit obtenu et le transforme éventuellement en un sel d'addition avec un acide.

5. Procédé selon la revendication 4, caractérisé en ce que la thionation s'effectue au moyen du bis(méthoxy-4 phényl)-2,4 dithioxo-2,4 dithia-1,3 diphospha-2,4 étanne.

6. Médicament, caractérisé en ce qu'il contient un produit selon la revendication 1, éventuellement en association avec un ou plusieurs diluants ou adjuvants compatibles et pharmaceutiquement acceptables.

**Revendications** pour l'Etat contractant: AT

1. Procédé de préparation d'un dérivé de la pyrrolidine de formule générale:

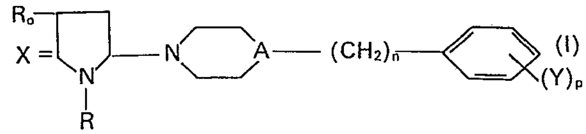

(I)

dans laquelle X représente un atome d'oxygène ou de soufre R représente un atome d'hydrogène ou un radical alcoyle, $R_o$ représente un atome d'hydrogène ou un radical alcoyle ou phényle éventuellement substitué par un atome d'halogène ou un radical alcoyle, alcoyloxy, alcoylthio ou nitro, A représente un atome d'azote ou un radical =CH−, les symboles Y, identiques ou différents, représentent un atome d'hydrogène ou d'halogène ou un radical alcoyle, alcoyloxy, alcoylthio, alcoylcarbonyle, nitro, amino, alcoylcarbonylamino, alcoylamino, dialcoylamino, alcoylsulfinyle, alcoylsulfonyle, alcoyloxycarbonyle, cyano, trifluorométhyle, hydroxy, mercapto, alcoylcarbonyloxy ou alcoylcarbonylthio et n représente le nombre 0 ou 1 et p représente le nombre 1, 2 ou 3, étant entendu que tous les radicaux alcoyle ou porteurs alcoyle sont en chaîne droite ou ramifiée et contiennent 1 à 4 atomes de carbone, ainsi que leurs sels d'addition avec les acides, caractérisé en ce que:

a) pour la préparation d'un produit de formule générale (I) dans laquelle X représente un atome d'oxygène et les autres symboles sont définis comme précédemment, on fait réagir un produit de formule générale:

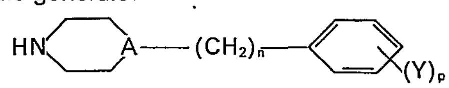

dans laquelle A, Y, n et p sont définis comme précédemment sur une pyrrolidinone-2 de formule générale:

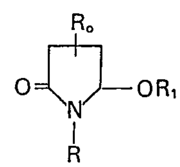

dans laquelle R et $R_o$ sont définis comme précédemment et $R_1$ représente un radical alcoyle, isole le produit attendu et le transforme éventuellement en un sel d'addition avec un acide, ou bien en ce que:

b) pour la préparation d'un produit de formule générale (I) dans laquelle $R_o$, A, Y, n et p sont définis comme précédemment, X représente un atome d'oxygène et R représente un radical alcoyle, on fait réagir un dérivé de formule générale:

R'Z

dans laquelle R' représente un radical alcoyle et Z représente un atome d'halogène ou un reste d'ester réactif sur un produit de formule générale:

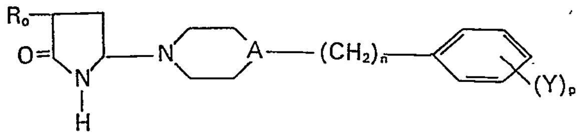

dans laquelle $R_o$, A, Y, n et p sont définis comme précédemment, puis isole le produit obtenu et le transforme éventuellement en un sel d'addition avec un acide, ou bien en ce que:

c) pour la préparation d'un produit de formule générale (I) dans laquelle X représente un atome de soufre et les autres symboles sont définis comme précédemment, on transforme par thionation un composé de formule générale (I) dans laquelle X représente un atome d'oxygène, c'est-à-dire un composé de formule générale:

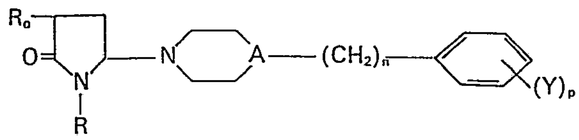

puis isole le produit obtenu et le transforme éventuellement en un sel d'addition avec un acide.

2. Procédé selon la revendication 1c, caractérisé en ce que la thionation s'effectue au moyen du bis(méthoxy-4 phényl)-2,4 dithioxo-2,4 dithia-1,3 diphospha-2,4 étanne.

**Claims** for the Contracting States BE, CH, DE, FR, GB, IT, LI, LU, NL, SE

1. Pyrrolidine derivative, characterised in that it corresponds to the general formula:

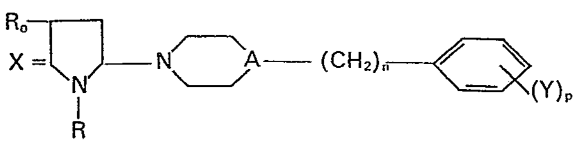

in which X denotes an oxygen or sulphur atom, R denotes a hydrogen atom or an alkyl radical, $R_o$ denotes a hydrogen atom or an alkyl radical, or a phenyl radical optionally substituted with a halogen atom or an alkyl, alkyloxy, alkylthio or nitro radical, A denotes a nitrogen atom or a radical =CH−, the symbols Y, which may be identical or different, denote a hydrogen or halogen atom or an alkyl, alkyloxy, alkylthio, alkylcarbonyl, nitro, amino, alkylcarbonylamino, alkylamino, dialkylamino, alkylsulphinyl, alkylsulphonly, alkyloxycarbonyl, cyano, trifluoromethyl, hydroxy, mercapto, alkylcarbonyloxy or alkylcarbonylthio radical, n denotes the number 0 or 1 and p denotes the number 1, 2 or 3, on the understanding that all the alkyl radicals or alkyl portions are linear- or branched-chain and contain 1 to 4 carbon atoms, as well as their addition salts with acids.

2. Process for preparing a product according to Claim 1, in the formula of which X denotes an oxygen atom and the other symbols are defined as in Claim 1, characterised in that a product of general formula:

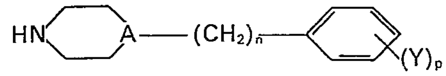

in which A, Y, n and p are defined as in Claim 1, is reacted with a 2-pyrrolidinone of general formula:

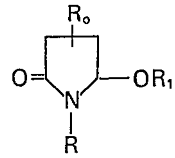

in which R and $R_o$ are defined as in Claim 1 and $R_1$ denotes an alkyl radical, and the expected product is isolated and optionally converted to an addition salt with an acid.

3. Process for preparing a product according to Claim 1, in the formula of which $R_o$, A, Y, n and p are defined as in Claim 1, X denotes an oxygen atom and R denotes an alkyl radical, characterised in that a derivative of general formula:

R'Z

in which R' denotes an alkyl radical and Z denotes a halogen atom or a reactive ester residue, is reacted with a product of general formula:

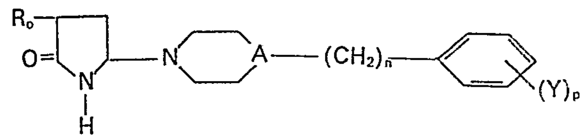

in which $R_o$, A, Y, n and p are defined as in Claim 1, and the product obtained is then isolated and optionally converted to an addition salt with an acid.

4. Process for preparing a product according to Claim 1, in the formula of which X denotes a sulphur atom and the other symbols are defined as in Claim 1, characterised in that a compound according to Claim 1 in the formula of which X denotes an oxygen atom, that is to say a compound of general formula:

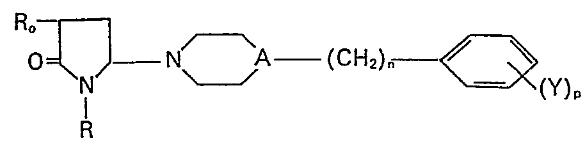

is converted by thionation, and the product obtained is then isolated and optionally converted to an addition salt with an acid.

5. Process according to Claim 4, characterised in that the thionation is accomplished by means of 2,4-bis-(4-methoxyphenyl)-2,4-dithioxo-1,3-dithia-2,4-diphosphetane.

6. Drug, characterised in that it contains a product according to Claim 1, optionally in combination with one or more diluents or adjuvants which are compatible and pharmaceutically acceptable.

**Claims** for the Contracting State: AT

1. Process for preparing a pyrrolidine derivative of general formula:

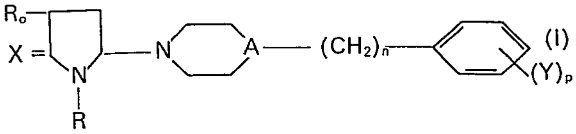

(I)

in which X denotes an oxygen or sulphur atom, R denotes a hydrogen atom or an alkyl radical, $R_0$ denotes a hydrogen atom or an alkyl radical, or a phenyl radical optionally substituted with a halogen atom or an alkyl, alkyloxy, alkylthio or nitro radical, A denotes a nitrogen atom or a radical $=CH-$, the symbols Y, which may be identical or different, denote a hydrogen or halogen atom or an alkyl, alkyloxy, alkylthio, alkylcarbonyl, nitro, amino, alkylcarbonylamino, alkylamino, dialkylamino, alkylsulphinyl, alkylsulphonyl, alkyloxycarbonyl, cyano, trifluoromethyl, hydroxy, mercapto, alkylcarbonyloxy or alkylcarbonylthio radical, n denotes the number 0 or 1 and p denotes the number 1, 2 or 3, on the understanding that all the alkyl radicals or alkyl portions are linear- or branched-chain and contain 1 to 4 carbon atoms, as well as their addition salts with acids, characterised in that,

(*a*) for the preparation of a product of general Formula (I), in which X denotes an oxygen atom and the other symbols are defined as above, a product of general formula:

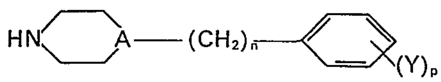

in which A, Y, n and p are defined as above, is reacted with a 2-pyrrolidinone of general formula:

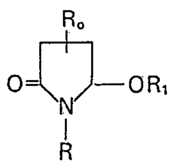

in which R and $R_0$ are defined as above and $R_1$ denotes an alkyl radical, and the expected product is isolated and optionally converted to an addition salt with an acid, or alternatively in that,

(*b*) for the preparation of a product of general Formula (I), in which $R_0$, A, Y, n and p are defined as above, X denotes an oxygen atom and R denotes an alkyl radical, a derivative of general formula:

R'Z

in which R' denotes an alkyl radical and Z denotes a halogen atom or a reactive ester residue, is reacted with a product of general formula:

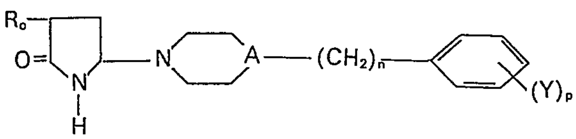

in which $R_0$, A, Y, n and p are defined as above, and the product obtained is then isolated and optionally converted to an addition salt with an acid, or alternatively in that,

(*c*) for the preparation of a product of general Formula (I) in which X denotes a sulphur atom and the other symbols are defined as above, a compound of general Formula (I) in which X denotes an oxygen atom, that is to say a compound of general formula:

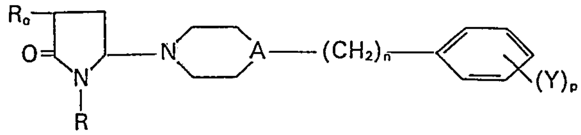

is converted by thionation, and the product obtained is then isolated and optionally converted to an addition salt with an acid.

2. Process according to Claim 1c, characterised in that the thionation is accomplished by means of 2,4-bis-(4-methoxyphenyl)-2,4-dithioxo-1,3-dithia-2,4-diphosphetane.

**Patentansprüche** für die Vertragsstaaten BE, CH, DE, FR, GB, IT, LI, LU, NL, SE

1. Derivat des Pyrrolidins, dadurch gekennzeichnet, dass es der allgemeinen Formel

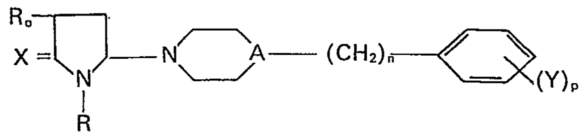

entspricht, worin X ein Sauerstoff- oder Schwefelatom bedeutet, R ein Wasserstoffatom oder einen Alkylrest bedeutet, $R_0$ ein Wasserstoffatom oder einen Alkyl- oder Phenylrest, der ggf. durch ein Halogenatom oder einen Alkyl-, Alkyloxy-, Alkylthio- oder Nitrorest substituiert ist, bedeutet, A ein Stickstoffatom oder einen Rest $=CH-$ bedeutet, die Symbole Y, die identisch oder verschieden sind, ein Wasserstoff- oder Halogenatom oder einen Alkyl-, Alkyloxy-, Alkylthio-, Alkylcarbonyl-, Nitro-, Amino-, Alkylcarbonylamino-, Alkylamino-, Dialkylamino-, Alkylsulfinyl-, Alkylsulfonyl-, Alkyloxycarbonyl-, Cyano-, Trifluormethyl-, Hydroxy-, Mercapto-, Alkylcarbonyloxy- oder Alkylcarbonylthiorest bedeuten, und n die Zahl 0 oder 1 bedeutet und p die Zahl 1, 2 oder 3 bedeutet, wobei alle Alkylreste oder Alkylteile in gerader oder verzweigter Kette stehen und 1 bis 4 Kohlenstoffatome enthalten, sowie ihre Additionssalze mit Säuren.

2. Verfahren zur Herstellung eines Produkts gemäss Anspruch 1, in dessen Formel X ein Sauerstoffatom bedeutet und die übrigen Symbole wie im Anspruch 1 definiert sind, dadurch gekennzeichnet, dass man ein Produkt der allgemeinen Formel

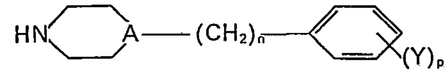

worin A, Y, n und p wie im Anspruch 1 definiert sind, auf ein Pyrrolidinon-2 der allgemeinen Formel

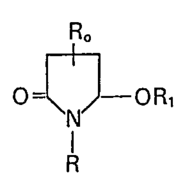

worin R und $R_0$ wie im Anspruch 1 definiert sind' und $R_1$ einen Alkylrest bedeutet, einwirken lässt, das erwartete Produkt isoliert und es ggf. in ein Additionssalz mit einer Säure überführt.

3. Verfahren zur Herstellung eines Produkts gemäss Anspruch 1, in dessen Formel $R_0$, A, Y, n und p wie im Anspruch 1 definiert sind, X ein sauerstoffatom bedeutet und R einen Alkylrest bedeutet, dadurch gekennzeichnet, dass man ein derivat der allgemeinen Formel

R'Z

worin R' einen Alkylrest bedeutet und Z ein Halogenatom oder einen Rest eines reaktiven Esters darstellt, auf ein Produkt der allgemeinen Formel

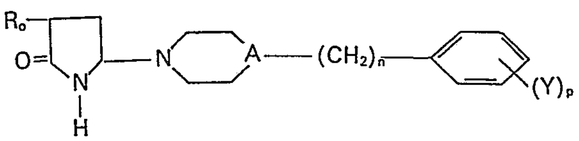

worin $R_0$, A, Y, n und p wie im Anspruch 1 definiert sind, einwirken lässt, das erhaltene Produkt dann isoliert und es ggf. in ein Additionssalz mit einer Säure überführt.

4. Verfahren zur Herstellung eines Produkts gemäss Anspruch 1, in dessen Formel X ein Schwefelatom bedeutet und die übrigen Symbole wie im Anspruch 1 definiert sind, dadurch gekennzeichnet, dass man eine Verbindung gemäss Anspruch 1, in deren Formel X ein Sauerstoffatom bedeutet, d.h. eine Verbindung der allgemeinen Formel

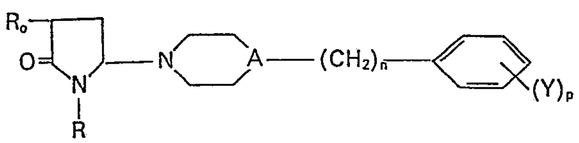

durch Thionierung umwandelt, dass man das erhaltene Produkt dann isoliert und es ggf. in ein Additionssalz mit Säure überführt.

5. Verfahren gemäss Anspruch 4, dadurch gekennzeichnet, dass die Thionierung mittels Bis-2,4-(4-methoxyphenyl)-2,4-dithioxo-1,3-dithia-2,4-diphosphaetan erfolgt.

6. Arzneimittel, dadurch gekennzeichnet, dass es ein Produkt gemäss Anspruch 1 enthält, ggf. in Verbindung mit einem oder mehreren verträglichen und pharmazeutisch annehmbaren Verdünnungs- oder Hilfsmitteln.

**Patentansprüche** für den Vertragsstaat: AT

1. Verfahren zur Herstellung eines Pyrrolidinderivats der allgemeinen Formel:

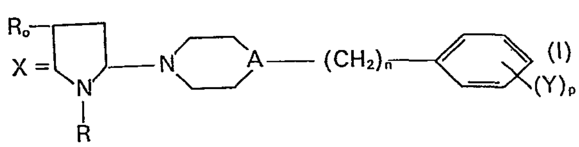

(I)

in welcher X ein Sauerstoff- oder Schwefelatom darstellt, R ein Wasserstoffatom oder einen Alkylrest darstellt, $R_0$ ein Wasserstoffatom oder einen Alkylrest oder Phenylrest, ggf. substituiert durch ein Halogenatom oder einen Alkyl-, Alkyloxy-, Alkylthio- oder Nitrorest, A ein Stickstoffatom oder einen Rest =CH– darstellt, die Symbole Y unabhängig voneinander ein Wasserstoff- oder Halogenatom oder einen Alkyl-, Alkyloxy-, Alyklthio-, Alkylcarbonyl-, Nitro-, Amino-, Alkylcarbonylamino-, Alkylamino-, Dialkylamino-, Alkylsulfinyl-, Alkylsulfonyl-, Alkoxycarbonyl-, Cyano-, Trifluormethyl-, Hydroxy-, Mercapto-, Alkylcarbonyloxy- oder Alkylcarbonylthiorest darstellen und n die Zahl 0 oder 1 bedeutet und p die Zahl 1, 2 oder 3 bedeutet, wobei alle Alkylreste oder Alkylteile selbtverständlich geradkettig oder verzweigt sind und 1 bis 4 Kohlenstoffatome enthalten, sowie von deren Säureadditionssalzen, dadurch gekennzeichnet, dass man

a) zur Herstellung einer Verbindung der allgemeinen Formel (I), worin X ein Sauerstoffatom darstellt und die übrigen Symbole die obige Bedeutung haben, eine Verbindung der allgemeinen Formel:

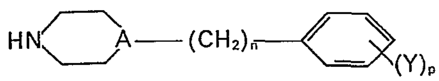

in welcher A, Y, n und p die obige Bedeutung haben, auf ein pyrrolidin-2-on der allgemeinen Formel

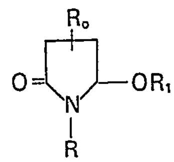

in welcher R und $R_0$ die obige Bedeutung haben und $R_1$ einen Alkylrest darstellt, einwirken lässt, das erwartete Produkt isoliert und es ggf. in ein säureadditionssalz überführt, oder dass man

b) zur Herstellung einer Verbindung der allgemeinen Formel (I), worin $R_0$, A, Y, n und p die obige Bedeutung haben, X ein Sauerstoffatom darstellt und R einen Alkylrest darstellt, ein Derivat der allgemeinen Formel:

R'Z

worin R' einen Alkylrest darstellt und Z ein Halogenatom oder einen reaktionsfähigen Ester darstellt, auf eine Verbindung der allgemeinen Formel:

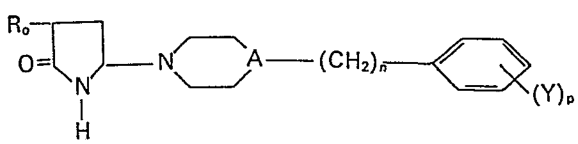

in welcher $R_0$, A, Y, n und p die obige Bedeutung haben, einwirken lässt, dann das erhaltene Produkt isoliert und es dann ggf. in ein Säureadditionssalz überführt, oder dass man

c) zur Herstellung einer Verbindung der allgemeinen Formel (I), worin X ein schwefelatom darstellt und die übrigen Symbole die obige Bedeutung haben, eine Verbindung der allgemeinen Formel (I), in welcher X ein sauerstoffatom bedeutet, d. h. eine Verbindung der allgemeinen Formel:

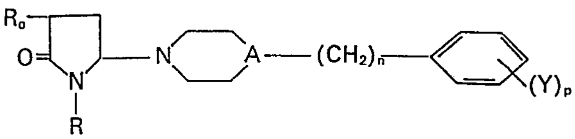

durch Thionierung umwandelt, dann das erhaltene Produkt isoliert und es ggf. in ein Säureadditionssalz überführt.

2. Verfahren nach Anspruch 1c, dadurch gekennzeichnet, dass die Thionierung mittels 2,4-Bis-(4-methoxyphenyl)-2,4-dithioxo-1,3-dithia-2,4-diphosphaetan erfolgt.